# EUROPEAN PATENT APPLICATION

(11) **EP 1 548 111 A1**
(43) Date of publication of application: **29.06.2005**
(21) Application number: 03797560.4
(22) Date of filing: 09.09.2003
(51) Int. Cl.: C12N 15/09, C12P 21/02, C12Q 1/00, G01N 33/15, G01N 33/50, G01N 33/53, G01N 33/566

(54) **PROCESS FOR PRODUCING LIGAND BINDING PROTEIN WITH THE USE OF CELL-FREE PROTEIN SYNTHESIS SYSTEM AND UTILIZATION THEREOF**

(30) Priority: 18.09.2002 JP 2002271375
(71) Applicant: RIKEN, Wako-shi, Saitama 351-0198 (JP)
(72) Inventor: KIGAWA, Takanori, c/o RIKEN Yokohama Institute, Yokohama-shi, Kanagawa 230-0045 (JP); MATSUDA, Takayoshi, c/o RIKEN Yokohama Institute, Yokohama-shi, Kanagawa 230-0045 (JP); AOKI, Masaaki, c/o RIKEN Yokohama Institute, Yokohama-shi, Kanagawa 230-0045 (JP); YOKOYAMA, Shigeyuki, c/o RIKEN Yokohama Institute, Yokohama-shi, Kanagawa 230-0045 (JP); SHINOZAKI, Kazuo, c/o RIKEN Yokohama Institute, Yokohama-shi, Kanagawa 230-0045 (JP); SEKI, Motoaki, c/o RIKEN Yokohama Institute, Yokohama-shi, Kanagawa 230-0045 (JP)
(74) Representative: Arends, William Gerrit
(86) International application number: PCT/JP2003/011474
(87) International publication number: WO 2004/027059

(57) **Abstract**

It is intended to provide a process for efficiently producing a ligand binding protein **characterized in that** this protein or its domain is synthesized in a cell-free protein synthesis system containing a ligand that binds to the protein or its domain. In a preferred embodiment, the above-described ligand is a substance selected from the group consisting of DNAs, RNAs, nucleosides, nucleotides, proteins, peptides, amino acids, lipids, carbohydrates, derivatives thereof, alkaloids, terpenes, low-molecular weight compounds, coenzymes, low-molecular weight metal complexes and metal ions, while the ligand binding protein or the ligand binding domain thereof is synthesized as a soluble protein.

## Description

### Technical Field

The invention relates to a method for producing a ligand-binding protein in a cell-free protein synthesis system. More specifically, the invention relates to a method for producing the ligand-binding protein in a cell-free protein synthesis system to which a ligand capable of binding to the ligand-binding protein is preliminarily added, and a method for screening a ligand and a method for screening a ligand-binding protein, using the production method, and the like.

### Background Art

Recently, DNA sequences of various organisms including human have been analyzed increasingly and rapidly. With regard to a large number of genes extracted from this enormous genome sequence information, it has been an important issue to systematically analyze three dimensional structures of proteins encoded by individual genes, and elucidate the relationship between structure and function thereof.

In this "Structural genomics", an important target among the proteins for their structural analysis is a group of ligand-binding proteins such as transcription factors, antibodies, receptor proteins and proteins involved in signal transductions in cells. Additionally, the structural determination and functional analysis of these proteins give useful information for the development of novel pharmaceutical products and the like.

In the method of related art for expressing such proteins at soluble states while the proteins form the accurate three dimensional structures, expression systems using cells such as *Escherichia coli,* yeast, animal cells and insect cells are widely used. However, some of the ligand-binding proteins exist stably or form their accurate three dimensional structures, only at their ligand-binding states. In case that an appropriate ligand is added to a liquid culture of viable cells and the like so as to express such proteins, the efficiency of cellular incorporation is generally low. Therefore, a large scale expression thereof involved much difficulty. In case that a protein needs a ligand to form its three dimensional structure (folding) or to maintain its folded state, it is difficult to achieve the purpose to form a normally folded state of the protein, even when the ligand is added to the protein that has been synthesized without any such ligand. In a cell-free system extremely useful as a protein synthesis system, further, a ligand-binding protein could not be expressed at a soluble state to form an accurate three dimensional structure.

Still further, the existence of numerous proteins unknown so far has been suggested through the analysis of genome nucleotide sequencing. Among these proteins, a great number of so-called orphan receptors have been found, which are similar to a ligand-binding protein such as a known receptor, but not only the functions of such receptors in vivo, but also ligands with which the receptors interact are unknown. As the method for analyzing the interactions of such proteins, so far, there is reported, for example, immunoprecipitation method (see for example non-patent reference 1), phage display method (see for example non-patent reference 2), yeast two-hybrid method (see for example patent reference 1 and non-patent reference 3), and protein chip method (see for example patent references 2 and 3). However, a method for screening a ligand capable of binding to a ligand-binding protein in a simple manner has been desired, where the method is applicable to any cases where the ligand is not only a protein but also a low molecular weight ligand.
[Patent Reference 1]
Specification of USP No. 5283173
[Patent Reference 2]
Official gazette of JP-A-2001-242116
[Patent Reference 3]
Specification of USP No. 6365418
[Non-patent Reference 1]
Phizicky, E. M., Fields, S., Microbiol. Rev., March 1995,
Vol. 59, p. 94-123
[Non-patent Reference 2]
Smith, G. P., Science, June 14, 1985, Vol. 228, p. 1315-1317
[Non-patent Reference 3]
Fields, S., Song, O., Nature, 1989, Vol. 340, p. 245

### Disclosure of the Invention

In such circumstances, it is an object of the present invention to provide a method for efficiently producing a ligand-binding protein. It is a further object of the present invention to provide a method for screening a ligand capable of binding to a ligand-binding protein, using the aforementioned production method. It is a still further object of the present invention to provide a method for screening a novel ligand-binding protein capable of binding to a specific ligand.

So as to achieve the above objects, the inventors made various investigations. Consequently, the inventors found that the above problems concerning the incorporation efficiency of ligand and protein decomposition can be overcome by synthesizing a ligand-binding protein in the presence of a ligand capable of binding to the ligand-binding protein using a cell-free protein synthesis system, to obtain a desired protein in a soluble state with the accurate three dimensional structure thereof, as well as a protein domain thereof. Thus, the invention has been completed.

In a first aspect, there is provided a method of producing a ligand-binding protein or a domain thereof in a cell-free protein synthesis system, to which a ligand capable of binding to the protein or the domain thereof is preliminarily added.

In a preferable embodiment, the ligand is a substance selected from the group consisting of DNAs, RNAs, nucleosides, nucleotides, proteins, peptides, amino acids, lipids, carbohydrates and derivatives thereof, alkaloids, terpenes, low molecular weight compounds, coenzymes, low molecular weight metal complexes and metal ions, while the ligand-binding protein or the ligand-binding domain thereof is synthesized as a soluble protein.

In a second aspect of the present invention, there is provided a method for screening a ligand capable of binding to a target protein, comprising the steps of (a) preparing a candidate ligand, (b) synthesizing the target protein in a cell-free protein synthesis system in the presence and absence of the candidate ligand, and (c) evaluating one or more properties selected from the group consisting of the synthetic rates, solubilities, specific activities, and the formations of higher structure of the resulting target protein under the respective conditions, where the method suggests that the candidate ligand binds to the target protein when the presence of the candidate ligand improves the synthetic rate and/or solubility of the target protein; the presence thereof improves or reduces the specific activity of the target protein; or the presence thereof leads to the formation of a desired higher structure. In accordance with the present invention, the reason of the increase of the synthetic rate (yield or amount) and/or the solubility of the target protein bound with the ligand probably lies in the stabilization of the three dimensional structure of the protein through the binding with the ligand, leading to the suppression of the decomposition thereof and also lies in the suppression of the insolubilization and aggregation of the protein due to the formation of the accurate three dimensional structure.

In a preferable embodiment, the target protein is a protein of unknown function or a polypeptide domain thereof, or the protein is a receptor, a transporter, a DNA-binding protein, a protein interactive with another protein, a cytoskeletal protein, an oxidoreductase, a chromoprotein or a ligand-binding domain thereof.

In one embodiment, the ligand capable of binding to the target protein is an activator or inhibitor of the target protein.

In a third aspect of the present invention, there is provided a method for screening a protein capable of binding to a target ligand, comprising the steps of (a) preparing a polynucleotide encoding a candidate protein, (b) expressing the polynucleotide in a cell-free protein system in the presence and absence of the target ligand, and (c) evaluating one or more properties selected from the group consisting of the synthetic rates, solubilities, specific activities, and the formations of higher structure of the resulting candidate protein under the respective conditions, where the method suggests that the candidate protein binds to the target ligand when the added target ligand improves or deteriorates one or more properties selected from the properties described above or when a desired higher structure is formed.

In a preferable embodiment, the target ligand is a substance selected from the group consisting of DNAs, RNAs, nucleosides, nucleotides, proteins, peptides, amino acids, lipids, carbohydrates and derivatives thereof, alkaloids, terpenes, low molecular weight compounds, coenzymes, low molecular weight metal complexes and metal ions, where the protein is a protein of unknown function or a polypeptide domain thereof. Furthermore, the invention provides a method for screening a novel target for drug creation by using a ligand with pharmacological activity or a protein known as a target for drug creation.

Herein, singular forms of terms used in this specification and the claims mean plural forms thereof, unless otherwise stated. For example, the term "protein" sometimes means one or two or more proteins.

### Brief Description of the Drawings

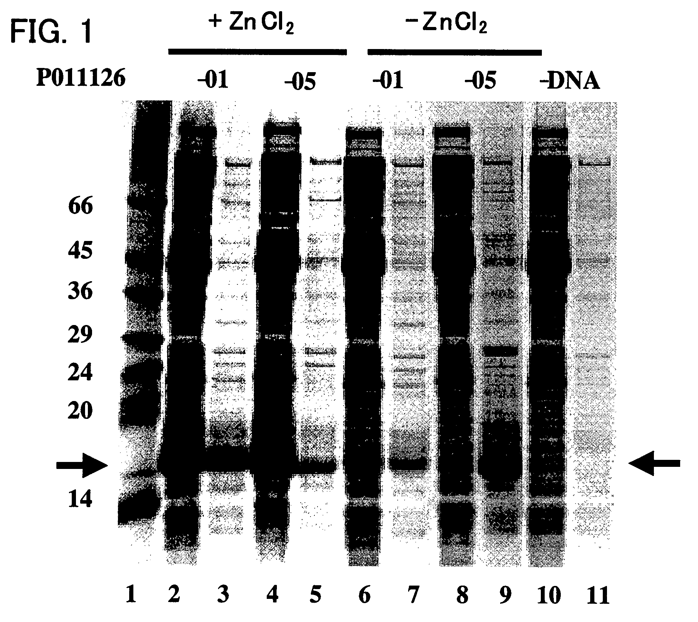

Fig. 1 is a photograph depicting the results of the Tricine-SDS-PAGE analysis of samples where two types of expression vectors P011126-01 and P011126-05 were expressed in the presence or absence of zinc chloride in a cell-free protein synthesis system. In the figure, the arrows indicate the expressed protein domains SPL9-DBD and SPL12-DBD, respectively.

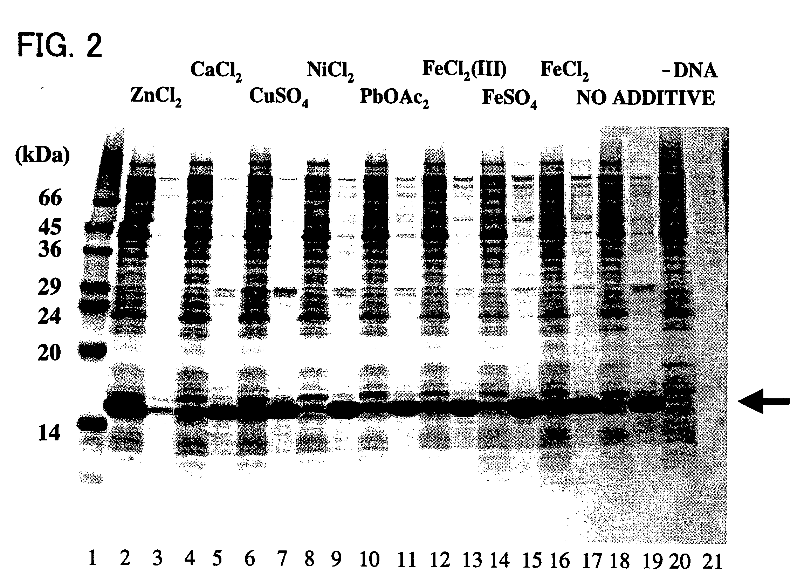

Fig.2 is a photograph depicting the results of the Tricine-SDS-PAGE analysis of a sample where an expression vector P011126-05 was expressed in the presence of various metal compounds in a cell-free protein synthesis system. In the figure, the arrow indicates the expressed protein domain SPL12-DBD.

### Best Mode for Carrying out the Invention

### (Ligand-binding protein)

It is known that most proteins in biological organisms form complexes to interact with specific ligands such as other proteins, DNAs, RNAs or low molecular weight compounds and low molecular weight metal complexes to exert their functions in metabolic pathway or signal transduction pathway. The proteins are, for example, specific antibodies against various antigens, receptors, GTP-binding proteins (G-proteins), metal-binding proteins, phosphorylated proteins, enzyme proteins for specific substrates, inhibitor proteins of enzymes, and DNA-binding proteins. In the embodiments of the invention, the term "low molecular weight compound" means a compound with a molecular weight of 1,000 or less, such as naturally occurring organic compounds and synthetic organic compounds. Additionally, the terms "low molecular weight metal complexes" or "metal complexes" mean, for example, porphyrin metal complexes and phthalocyanine metal complexes.

As the target for the development of pharmaceutical products, for example, G-protein-coupled receptor (GPCR), transporter, and ion channel capable of binding to low molecular weight ligands are important. The target includes, for example, adrenalin receptor, acetylcholine receptor, histamine receptor, dopamine receptor, serotonin receptor, glutamate receptor, serotonin transporter, estrogen receptor, Ca²⁺ channel, Na⁺ channel, and Cl⁻ channel. In the growth hormone receptor family with only one transmembrane domain, the receptor is extracellularly dimerized when bound with a ligand protein, so that the structure of the receptor is modified to cause the relative change of the structure of the transmembrane domain, leading to the modification of the structure of the intracellular domain. It is known that in GPCR having seven transmembrane domains and intracellular domain to which the G-protein binds, the structure of the transmembrane domains is markedly modified by the ligand binding, leading to a significant change in the loop structure that links these domains, so that the activation of the G protein is triggered.

Metal-binding proteins include, for example, transferrin and ferritin adjusting the amount of iron *in vivo,* and metallothionein capable of binding with heavy metals such as zinc. Additionally, among enzymes, many metal enzymes require metal ions for activity exertion and the stabilization of the structures. In one embodiment of the present invention, the metal species for use as a ligand may be dissolved in the reaction system even when it is at a low concentration and includes, for example, but is not limited to calcium, cadmium, zinc and iron.

Furthermore, the DNA-binding protein includes, for example, proteins capable of relatively strongly binding to chromosomal DNA to be involved in the retention of the higher structure of chromosome (for example, histone), proteins binding to double-stranded DNA to be involved in the expression and regulation of gene (for example, transcription factor), proteins capable of binding to double-stranded DNA or single-stranded DNA and having enzyme activities for cleavage and repair (for example, restriction enzyme), proteins capable of binding to single-stranded DNA to be involved in replication, repair, recombination or expression and regulation of some genes (for example, single-stranded DNA-binding protein), proteins catalyzing the change of DNA structure and topology (for example, DNA helicase).

Protein with protein-protein interaction includes, for example, various proteins for intracellular transduction of a signal from a ligand bound to the receptor described above and the like. For signal transduction from the membrane receptor, G-protein is one of particular importance. G-protein includes three peptides, namely α subunit capable of binding to GTP to hydrolyze GTP, and β and γ subunits forming a dimer strongly bound wi th non-covalent bond. When GDP binds to α subunit, the α subunit binds to the βγ complex, to form an inactive trimer, which binds to the C terminus of the receptor. When the ligand binds to the receptor, the structure of the cytoplasmic domain of the receptor changes. Continuously, the structure of the GDP-binding α subunit changes, so that the affinity with GDP is reduced to dissociate GDP from the active site of the α subunit. Because the intracellular GTP concentration is about 10 fold that of GDP, GTP immediately binds to the active site. The α subunit bound with GTP forms an activated structure, so that the α subunit is dissociated from both the receptor and the βγ complex to activate other effector molecules to intracellularly transmit the information from the receptor. The effector molecules to be activated by the G-protein include, for example, adenylate cyclase, phospholipase C (PLC), phospholipase A₂ (PLA₂), phosphoinositide 3-kinase (PI₃ kinase) and β-adrenergic receptor kinase (βARK).

The protein with protein-protein interaction also includes proteins involved in intracellular signal transduction mechanism. The intracellular signal transduction mechanism requires fundamental factors such as 1) serine/threonine kinase, tyrosine kinase, serine/threonine phosphatase, tyrosine phosphatase, 2) second messenger, 3) G proteins of α, β, γ subunit types, 4) low molecular weightGTP-binding protein. These fundamental factors essentially are modified into active type, upon receiving upstream signal. After transmitting the signal to the downstream, the factors are back again to inactive type. Such procedures are repeatedly carried out to transmit signal. Proteins involved in them include, for example, kinase A and kinase C as serine/threonine kinase, epidermal growth factor receptor and Src family member as tyrosine kinase, phosphatase 1, 2A, 2B and 2C as serine/threonine phosphatase, and CD45 and LAR as tyrosine phosphatase. Additionally, the G proteins of α, β, γ subunit types include Gs and Gq coupling receptors with adenylate cyclase and phospholipase C, while low molecular weight GTP binding proteins include Ras, Rho and Rabn.

Cytoskeletal proteins include tubulin forming microtubule, actin forming actin filament, and vimentin, keratin and lamin, etc. forming intermediate filament. These proteins bind to, for example, ATP and GTP to be associated with each other or with other proteins to form a cytoskeleton, so that the cell itself changes its shape or migrates or transfers the organelle (intracellular small organ) in cytoplasm or separates chromosome at the M phase.

Oxidoreductases include, for example, oxidoreductase and oxigenase and interact, for example, with coenzymes such as NAD and NADP, quinones and cytochrome. Chromoproteins includes, for example, opsin constituting visual dye, phytochrome as plant dye protein, phototropin, and phycocyanine as a convergent chromoprotein in relation with photosynthesis. These proteins interact with individual dyes as prosthetic groups in various binding modes (coordination bond, ion bond, hydrogen bond and the like).

### (Construction of polynucleotide for expression)

The ligand-binding protein or a ligand-binding domain thereof is expressed in a cell-free protein synthesis system containing a ligand capable of binding to the protein or the domain. The polynucleotide for the expression in the cell-free protein synthesis system may be prepared by appropriate methods having been known. The polynucleotide may be DNA or RNA. Additionally, the polynucleotide may be labeled with fluorescent substances, may be methylated, may have a cap structure added, and may be substituted with nucleotide analogs and the like.

DNA is generally double-stranded but may be circular double-stranded or linear double-stranded, and is transcribed and translated in the cell-free protein synthesis system. The method for preparing these template DNAs is well known to a person skilled in the art. Such template DNA may be prepared by recombinant DNA technique using *Escherichia coli* and the like as hosts. Otherwise, such template DNA may be prepared by *in vitro* DNA amplification technique such as PCR, without any transformation of host cell. RNA is generally used as single-stranded mRNA and is translated in the cell-free protein synthesis system. These techniques are described in, for example, Sambrook *et al*., Molecular Cloning: A Laboratory Manual, Second Edition, 1989; D. N. Glover (ed.), DNA Cloning, Volumes I and II, 1985; M. J. Gait (ed.), Oligonucleotide Synthesis, 1984, etc.

The sequence required for the transcription and translation in the cell-free synthesis system includes sequences of, for example, strong promoter such as T7 promoter, ribosome binding site, and T7 terminator. Additionally, the sequence of a fusion protein such as GST and the tag sequence such as histidines may be added for efficiently purifying expressed fusion proteins.

The target ligand-binding protein or a ligand-binding domain thereof may be expressed singly or as a fusion protein with another protein, or may be expressed as a protein with added tag. As another protein, for example, glutathione S-transferase (GST), maltose-binding protein (MBP), protein A, or protein G is used. As the tag, histidine tag (His tag) or Flag tag is used.

In a preferable embodiment of the invention, the ligand-binding protein or a ligand-binding domain thereof can be expressed as a fusion protein with thioredoxin. The thioredoxin for use in this embodiment includes not only an *E. coli* thioredoxin (Lunn, C. A. et al., J. Biol. Chem. 259, 10469-10474 (1984)) but also a mammalian thioredoxin (Wollman, E. E. et al., J. Biol. Chem. 263, 15506-15512 (1988)), an yeast thioredoxin, and a thioredoxin domain of protein disulfide isomerase (Edman, J. C. et al., Nature 317, 267-270 (1985)), as well as a polypeptides formed as a result of deletion, addition or substitution of one or plural amino acids in a polypeptide listed above which can catalyze the formation of disulfide bond.

Various methods can be used for expressing the target ligand-binding protein as a fusion protein with thioredoxin. For example, thioredoxin is fused to the N terminus or C terminus of the ligand-binding protein or the thioredoxin sequence is inserted between the domains constituting the protein, to express the fusion protein. A plasmid vector carrying a multi-cloning site in the vicinity of the thioredoxin sequence is preferably used for preparing a template DNA in a cell-free protein synthesis system, by inserting the gene of the target ligand-binding protein. To isolate only the target ligand-binding protein from the fusion protein by limited digestion, a specific sequence to be cleaved with protease such as enterokinase is particularly preferably inserted in the interface region with thioredoxin.

### (Protein synthesis in cell-free protein synthesis system)

A polynucleotide prepared as described above is expressed in a cell-free protein synthesis system. The cell-free protein synthesis system according to the invention is a system in which a protein is synthesized *in vitro* using a cell extract, and such a system may be a cell-free translation system in which information of mRNA is read and a protein is synthesized on a ribosome, or a system involving both of a cell-free transcription system and a cell-free translation system in which DNA is employed as a template to synthesize RNA. When using DNA as a template, an amplification reaction in vitro such as PCR allows a large number of template DNAs to be prepared simultaneously and rapidly without employing a complicated procedure such as a cloning required conventionally.

A cell extract mentioned above may be an extract solution from a eukaryotic or prokaryotic cell containing components required for synthesizing a protein, such as ribosome and tRNA. Any known eukaryotic or prokaryotic cell can be employed, including *E.coli*, thermophilic bacteria, wheat germ, rabbit reticulocyte, mouse L cell, Ehrlich's ascitic cancer cell, HeLa cell, CHO cell and budding yeast and the like, with an *E.coli*-derived one (such as *E.coli* S30 cell extract) or highly thermophilic bacteria (Thermus thermophilus) -derived one being preferred for the purpose of obtaining a high synthetic yield. Such an *E. coli* S30 cell extract can be prepared from *E. coli* A19 (*rna, met*), BL21, BL21 star, BL21 codon plus strains and the like by a known method (see, Pratt, J.M. et al., Transcription and translation - apractical approach, (1984), pp.179-209, Henes, B.D. and Higgins, S.J. ed., IRL Press, Oxford), or it may be any of commercially available ones supplied from Promega or Novagen. Various compositions and methods of the cell-free protein synthesis system have been developed. For example, JP-A-2000-175695 describes in detail the highly efficient synthesis system using a dialysis method modified by the inventors. The contents thereof are incorporated herein by reference in their entireties.

By the method of the invention, a ligand capable of binding to a protein or a domain thereof is added to the cell-free protein synthesis system. The ligand varies depending on the protein or the domain thereof to be expressed. The ligand is any one or more types of ligands selected from the group consisting of DNAs, RNAs, nucleosides, nucleotides, proteins, peptides, amino acids, lipids, carbohydrates and derivatives thereof, alkaloids, terpenes, low molecular weight compounds, coenzymes, low molecular weight metal complexes and metal ions. The amount of the ligand to be added is an amount equivalent to one- to 10-fold the amount of the calculated amount of a synthesized protein in the synthesis system. Such ligand is added at a concentration of 1 µM to 100 mM, preferably 1 µM to 1000 µM.

The synthetic rate (yield) of the protein or the domain thereof expressed in the cell-free protein synthesis system can be assayed by various methods. SDS polyacrylamide gel electrophoresis (SDS-PAGE) is the most common analysis method. When a protein is treated with SDS under reducing conditions, the protein is completely denatured and dissociated into polypeptides. The resultingpolypeptide binds to SDS at an amount about 1.4-fold by weight under reducing conditions, to form an SDS complex. The SDS complex thus formed migrates at a constant speed in a constant electric field due to the negative charge of excessive dodecyl sulfate ions. Accordingly, electrophoresis on SDS- containing polyacrylamide gel can separate protein, based on the molecular weight as a result of the molecular sieving action, so that a target protein can be detected, using the molecular weight thereof as an index. SDS-PAGE is classified into a continuous buffer system employing a buffer solution at a single pH and a non-continuous buffer system employing a buffer solution at plural pHs. Depending on the type of a protein to be separated, additionally, electrophoresis on a gel at low BIS concentration, gradient-gel electrophoresis, tricine-SDS-PAGE and the like can be used. The separated protein can generally be stained and assayed with Coomassie Blue. By silver staining method, the protein can be identified at sensitivity 20- to 100-fold that ofCoomassie bluestaining. Otherwise, the protein can be detected highly sensitively on gel, using commercially available fluorescent reagents such as SYPRO Ruby and SYPRO Orange (Patton, W. F. Electrophoresis, 21, 1123-1144 (2000)). Additionally, the protein when labeled with radioactivity can be detected by autoradiography and fluorography.

Further, other methods for detecting a target protein using the molecular weight as the marker include various methods known to a person skilled in the art, such as gel filtration chromatography, capillary electrophoresis and mass spectrometry but not limited thereto. Concerning mass spectrometry, see for example Yates, JR., III, J. Mass Spectrum. 1998, Vol. 33, pp. 1-19 about the principle of protein analysis by ionization and mass spectrometry and the protein identification using MALDI (matrix-assisted laser desorption ionization) and electrospray ionization (ESI).

### (Method for screening a ligand capable of binding to target protein)

So-called "orphan receptor" with no essential ligand having been elucidated yet exists among receptors. In accordance with the invention, a method of screening a ligand is provided, including preparing a candidate ligand of such target protein, synthesizing the target protein in the presence and absence of the candidate ligand in a cell-free protein synthesis system, and evaluating the synthetic rate, solubility, specific activity and/or the formation of the higher structure of the target protein synthesized under the respective conditions, where the method suggests that the candidate ligand binds to the target protein when the presence of the candidate ligand improves the synthetic rate and solubility of the target protein, improves or reduces the specific activity thereof and/or allows the formation of desired higher structure of the target protein. In accordance with the invention, the term "synthetic rate" means any of the amount of the protein synthesized per unit volume of the cell-free protein synthesis system or the amount of the protein synthesized per unit time. Additionally, the term "solubility" means the amount of the protein to be recovered in a soluble fraction among the total proteins synthesized in the cell-free synthesis system. Herein, various ligands may be suggested as the candidate ligand and include, for example, substances selected from the group consisting of DNAs, RNAs, nucleosides, nucleotides, proteins, peptides, amino acids, lipids, carbohydrates and derivatives thereof, alkaloids, terpenes, low molecular weight compounds, coenzymes, low molecular weight metal complexes and metal ions. A compound or molecule belonging to them is added singly or in combination of two or more thereof to the cell-free protein synthesis system. The synthetic rate and solubility of such protein can be assayed with SDS-PAGE or Tricine-SDS-PAGE.

In a preferable embodiment, the synthetic rate and solubility of such protein in the cell-free protein synthesis system can be assayed by preparing a fusion protein with a reporter protein. For example, the β-galactosidase gene (*lac*Z) derived from *Escherichia coli* is frequently used as a reporter gene in the transformation of various cells. The substrate of the enzyme *in viv*o is lactose. The enzyme can hydrolyze almost all types of β-galactoside. For example, ONPG as a colorless substrate is hydrolyzed to yellow o-nitrophenol with optical absorption at 420 nm. Additionally, x-gal is decomposed with the enzyme to exert the chromogenic reaction of indigo blue.

Alkaline phosphatase is an enzyme with extremely high catalytic activities of molecules. Because various fluorescent and luminous substrates have been developed therefor, the enzyme is preferable for highly sensitive assay.

Firefly luciferase catalyzes the oxidation of luciferin in the presence of ATP. Accordingly, the luciferase catalyzes the chemiluminescence reaction generating photons. Generally, sharp spike-wave light is radiated and rapidly reduced. When coenzyme A is added to the reaction system, more uniform luminescence can be obtained.

In the embodiment, green fluorescent protein (GFP) is particularly preferably used. GFP is a fluorescent protein discovered in Aequorea victoria and the like. It is known that the protein energetically transform the blue chemiluminescence of aequorin that concurrently exists as a luminescent protein, to green (Prasher, D., et al., Gene 11, 229-233, 1992). The GFP can automatically form a fluorophore on the apo-protein thereof without any requirement of a specific fluorophore, as is required by aequorin and luciferase. Thus, so far, GFP has been expressed in bacteria, yeast, fungal cells, plant cells, insect cells and animal cells using cloned GFP cDNA, for use as a marker of gene expression. Additionally, several mutations are introduced into the GFP gene by various methods, to prepare numerous GFP derivatives. It has been reported that some of the derivatives among them can exert higher fluorescent intensities than those of the wild type GFP or can emit fluorescence of different wavelengths (see Ehrig, T., O'Kane, D., and Prendergast, F. FEBS Lett. 367, 163-166, 1995; and Crameri, A., Whitehom, E., Tate, E., and Stemmer, W. Nature Biotech. 14, 315-319, 1996). Even in the embodiment, the synthetic rate of the protein in the cell-free protein synthesis system can be assayed rapidly in a simple manner by using these GFP types or derivatives thereof.

In another embodiment of the invention, additionally, the specific activity of the target protein or the formation of the higher structure thereof canbe evaluated instead of the synthesis rate and solubility of the target protein, to identify whether or not the protein can bind to the candidate ligand. General biochemical assay methods of activity are used in a manner dependent on the characteristic properties of each protein, as the assay method of the activity of the target protein. Alternatively, various methods can be used for evaluating the formation of the higher structure of protein. Herein, the term "higher structure" means, for example, secondary structures including α-helix, β-sheet structure and random coil, tertiary structure prepared by folding such secondary structure in space, and quaternary structure formed by the accumulation of plural such units as subunit components in some protein. Desired higher structure means any of the higher structures as required for the protein to exert the functions *in vivo.* These higher structures can be analyzed, using spectrometric methods such as circular dichroism, X-ray crystal structure analysis and nuclear magnetic resonance (NMR). NMR is widely applied as an analytical method of the three dimensional structures of biological polymers such as protein and nucleic acid, together with X-ray crystal structure analysis method. NMR analysis is preferably carried out for molecules with molecular weights of 20,000 to 30,000 or less. Such molecules are appropriate for the ligand-binding domains as a unit. NMR signal sharply reflects the local environmental change of molecules (binding with ligand, structural change, etc.). When protein binds with ligand to form a complex, for example, NMR signal can specifically detect the structure of the protein at the binding state to the ligand. When ¹⁵N-¹H HSQC spectrum using a protein uniformly labeled with ¹⁵N is used, only the NMR signal of the protein can be observed. For the analysis of protein-protein interaction, for example, the change in the chemical shift of NMR signal following the complex formation (chemical shift perturbation method), the change of hydrogen-deuterium substitution velocity in the amide hydrogen in main chain (H-D exchange method), and cross saturation method (see Kazuo Shimada, Protein, Nucleic Acid, Enzyme, 2002, Vol.47, No. 10, pp. 1285-1291) can additionally be used.

Specific examples of such ligand-binding protein include receptors, transporters, DNA-binding proteins, proteins with protein-protein interaction, cytoskeletal proteins, oxidoreductases, chromoproteins or ligand-binding domains thereof. It is known that when the binding of a receptor and a ligand is abnormal, a biologically diseased condition emerges. Screening a substance that activates a receptor more strongly than the essential ligand, or screening a molecule that inhibits receptor-ligand binding, is a very important method for the development of pharmaceutical product. By the method of the invention, an activating agent or an inhibitor of the target protein such as receptor can be screened.

### (Method for screening protein binding to target ligand)

In case that protein such as receptor for a specific ligand is unknown, a method for screening a protein capable of binding to a target ligand is provided, including preparing a polynucleotide encoding a candidate protein for the target ligand, expressing the polynucleotide in the presence and absence of the target ligand in a cell-free protein synthesis system, and evaluating one or more properties selected from the group consisting of the expression rates, solubilities, specific activities and the formations of the higher structure of the candidate protein expressed under the respective conditions. When one or more of the properties are improved or deteriorated due to the addition of the target ligand or when a desired higher structure is formed, the screening method suggests that the candidate protein binds to the target ligand. In accordance with the invention, the target ligand includes DNAs, RNAs, nucleosides, nucleotides, proteins, peptides, amino acids, lipids, carbohydrates and derivatives thereof, alkaloids, terpenes, low molecular weight compounds, co-enzymes, low molecular weight metal complexes and metal ions and also includes physiologically active substances or drugs belonging to them. For example, recently, it has been known that various immunosuppressants bind to intracellular specific receptors, to prepare complexes and that the resulting complexes inhibit the activation of a transcription factor mediating the signal transduction pathway, to suppress the activation of T cell as an immune cell. The method of the invention can be used for drugs such as immunosuppressants with unknown mechanisms, to screen a receptor to which these drugs can bind directly and to elucidate the action mechanism, leading to the development of a novel pharmaceutical product.

### Examples

The results of the investigation of the methods according to the present invention are explained in more detail by the following Examples using protein domains SPL9-DBD derived from *Arabidopsis thaliana* [the 66-151 amino acid residues (SEQ ID No. 2) encoded by *Arabidopsis* cDNA RAFL04-13-I11 (see GenBank Ac. No. AY046007)] and SPL12-DBD [the 124-205 amino acid residues (SEQ ID NO. 4) encoded by *Arabidopsis* cDNA RAFL09-41-M16 (see GenBank Ac. No. AY091024)]. It will possibly be suggested that these protein domains may have DNA-binding activity and zinc-binding activity, based on the homology analysis thereof with other DNA-binding proteins and the zinc finger protein in terms of amino acid sequence, using homology retrieval programs such as BLAST. However, the invention is not limited to these

### Examples.

### [Example 1]

### (1) Construction of expression vectors

Forgeneexpressioninacell-freeproteinsynthesissystem, DNA fragments were prepared using the two-step polymerase chain reaction (two-step PCR) method (see International Publication WO 03/004703), where T7 promoter, SD sequence and T7 terminator were added to the SPL9-DBD and SPL12-DBD genes.

### (Case of SPL9-DBD)

A first PCR was carried out, using a liquid recombinant *Escherichia coli* culture (used as 10-fold dilution) containing a plasmid where cDNA RAFL04-13-I11 encoding SPL9-DBD was cloned in the plasmid pBluescript SK+, and the following primers:

5′ primer: 5′-GGTGCCACGCGGATCCGGTCAGTCGGGTCAG-3′
(atr001003851.66FW; SEQ ID No.5); and

3′ primer: 5′-GGGCGGGATCAATCAATCATTACGCAGGCTGTGGCT-3′
(atr001003851.151RV; SEQ ID No.6).

Table 1 shows the composition of the PCR reaction mixture, while Table 2 shows the PCR reaction program.

**[Table 1]**

| Composition of primary PCR reaction solution | | | |
|---|---|---|---|
| Composition | Concentration | Amount added | Final concentration |
| Recombinant *E. coli* culture containing template plasmid | (×1/10) | 3 µl | (×3/200) |
| 5' primer atr001003851.66FW | 0.25 µM | 4 µl | 0.05 µM |
| 3' primer atr001003851.151RV | 0.25 µM | 4 µl | 0.05 µM |
| dNTPs (Toyobo) | 2 mM | 2 µl | 0.2 mM |
| Expand HiFi buffer (containing 15 mM magnesium chloride) (Roche) | (10×) | 2 µl | (1×) |
| Sterile distilled water | | 4.85 µl | |
| DNA polymerase (Roche) | 3.5 U/µl | 0.15 µl | 0.02625 U/µl |
| Total | | 20 µl | |

**[Table 2]**

| Programs of primary and secondary PCR reactions | | |
|---|---|---|
| STEP 1 | 94 °C | 30 sec |
| STEP 2 | 60 °C | 30 sec |
| STEP 3 | 72 °C | 2 min |
| STEP 4 | GOTO 1 for 9 times | |
| STEP 5 | 94 °C | 30 sec |
| STEP 6 | 60 °C | 30 sec |
| STEP 7 | 72 °C | 2 min + 5 sec/cycle |
| STEP 8 | GOTO 5 for 19 times | |
| STEP 9 | 72 °C | 7 min |
| STEP 10 | 4 °C | |

Then, a second PCR was carried out, using the first PCR product obtained through the reaction (used as 5-fold dilution) and the following primers: 
5′ primer with His tag sequence at downstream of T7 promoter:

5′-GCTCTTGTCATTGTGCTTCGCATGATTACGAATTCAGATCTCGATCCCGCGAAATT

AATACGACTCACTATAGGGAGACCACAACGGTTTCCCTCTAGAAATAATTTTGTTTAAC

TTTAAGAAGGAGATATACATATGAAAGATCATCTCATCCACAATGTCCACAAAGAGGAG

CACGCTCATGCCCACAACAAGAGCTCTGGCGCCTCCCTGGTGCCACGCGGATCC-3′
 (T7P-DM2-NHis; SEQ ID No. 7); 
3′ primer with T7 terminator sequence:

5′-AATGATTGATTGATCCCCGCCCAGCTGAGTTGGCTGCTGCCACCGCTGAGCAATAA

CTAGCATAACCCCTTGGGGCCTCTAAACGGGTCTTGAGGGGTTTTTTGCTGAAAGGAGG

AACTATATCCGGATAACCTCGAGCTGCAGGCATGCAAGCTTGGCGAAGCACAATGACAA

GAGC-3′ (T7T-DM5; SEQ ID No. 8): and
 universal primer: 5'-GCTCTTGTCATTGTGCTTCG-3'(U2; SEQ ID No. 9). Table 3 shows the composition of the PCR reaction mixture. Herein, the program shown in Table 2 was used as the PCR reaction program.

**[Table 3]**

| Composition of secondary PCR reaction solution | | | |
|---|---|---|---|
| Composition | Concentration | Amount | Final |
| First PCR product (template) | (×1/5) | 5 µl | (×1/20) |
| 5' primer T7P-DM2-NHis | 2 µM | 0.5 µl | 0.05 µM |
| 3' primer T7T-DM5 | 2 µM | 0.5 µl | 0.05 µM |
| Universal primer: U2 | 100 µM | 0.2 µl | 1 µM |
| dNTPs (Toyobo) | 2 mM | 2 µl | 0.2 mM |
| Expand HiFi buffer (containing 15 mM magnesium chloride) (Roche) | (10×) | 2 µl | (1×) |
| Sterile distilled water | | 9.65 µl | |
| DNA polymerase (Roche) | 3.5 U/µl | 0.15 µl | 0.02625 U/µl |
| Total | | 20 µl | |

### (Case of SPL12-DBD)

A first PCR was carried out, using a liquid recombinant *Escherichia coli* culture (used as 10-fold dilution) containing a plasmid where cDNA RAFL09-41-M16 encoding SPL12-DBD was cloned in the plasmid pBluescriptIISK+, and the following primers: 5' primer: 5'-GGTGCCACGCGGATCCGCGATTTGTTGTCAGGTAG-3' (atr001004282.124FW; SEQ ID No. 10); and 
3′ primer: 5′-GGGCGGGATCAATCAATCATTAAGTATCAGGATTTGCTTTTCT-3′

(atr001004282.205RV; SEQ ID No. 11).
 The composition of the PCR reaction mixture and the PCR reaction program in Tables 1 and 2, respectively are used as the composition of the PCR reaction mixture and the PCR reaction program (herein, the 5' primer and 3' primer are as follows:
5' primer: atr001004282.124FW and 3' primer: atr001004282.205RV) . Furthermore, a second PCR was carried out, using the first PCR product obtained through the reaction and the 5' primer with the His tag sequence downstream the T7 promoter (T7P-DM2-NHis; SEQ ID No. 7), 3' primer with the T7 terminator sequence (T7T-DM5; SEQ ID No. 8) and universal primer (U2; SEQ ID No. 9).
The composition of the PCR reaction mixture and the program in Tables 3 and 2 respectively are used.

Continuously, the DNA fragments were cloned into pPCR2.1 with TOPO TA-cloning kit (Invitrogen), to construct expression vectors P011126-01 (SPL9-DBD) and P011126-05 (SPL12-DBD).

### (2) Cell-free protein synthesis using dialysis method

*E. coli* S30 extract was prepared from *E. coli* strain BL21 codon plus according to the method of Zubay, *et al.* (Annu. Rev. Geneti., 7, 267-287, 1973). The protein synthesis reaction was performed by dialyzing a reaction solution of the composition shown below in Table 4 against a dialysis solution of the composition in Table 5, at 30°C for 8 hours. The reaction scale was as follows; zinc or other metal compounds were added both to 30µl of the reaction solution and 300µl of the dialysis solution at a final concentration of 50µM. At a control test, the synthesis reaction was done without these metal compounds added.

**[Table 4]**

| Composition of protein synthesis reaction | |
|---|---|
| Composition | Concentration |
| HEPES-KOH, pH 7.5 | 58.0 mM |
| Dithiothreitol (DTT) | 1.8 mM |
| ATP | 1.2 mM |
| CTP, GTP, UTP | 0.8 mM each |
| Creatine phosphate | 80.0 mM |
| Creatine kinase | 0.25 mg/ml |
| Polyethylene glycol 8000 | 4.0 % |
| 3', 5'-cAMP | 0.64 mM |
| L(-)-5'-Formyl-5,6,7,8-tetrahydrofolic acid | 68 µM |
| *E. coli* total tRNA | 175 µg/ml |
| Potassium glutamate | 210.0 mM |
| Ammonium acetate | 27.5 mM |
| Magnesium acetate | 10.7 mM |
| Amino acids (20 types) | 1.5 mM each |
| Sodium azide | 0.05 % |
| T7 RNA polymerase | 66.6 µg/ml |
| *E. coli* S30 extract | 30 vol. % of the reaction mixture |
| Template DNA (expression vector P011126-01 or | 1 µg/ml |
| expression vector P011126-05) | |

**[Table 5]**

| Composition of dialysis solution | |
|---|---|
| Composition | Concentration |
| HEPES-KOH, pH 7.5 | 58.0 mM |
| Dithiothreitol (DTT) | 1.8 mM |
| ATP | 1.2 mM |
| CTP, GTP, UTP | 0.8 mM each |
| Creatine phosphate | 80.0 mM |
| Creatine kinase | 0.25 mg/ml |
| Polyethylene glycol 8000 | 4.0 % |
| 3', 5'-cAMP | 0.64 mM |
| L(-)-5'-Formyl-5,6,7,8-tetrahydrofolic acid | 68 µM |
| Potassium glutamate | 210.0 mM |
| Ammonium acetate | 27.5 mM |
| Magnesium acetate | 14.9 mM |
| Amino acids (20 types) | 1.5 mM each |
| Sodium azide | 0.05 % |
| Tris-acetate (pH 8.2) | 3 mM |
| Potassium acetate | 18 mM |

### (3) Evaluation of synthetic rates and solubilities

50µl of 1×PBS buffer (137mM NaCl, 8.1mM NaH₂PO₄, 2.68mM KCl, 1.47mM KH₂PO₄, pH 7.8) was added to 10µl of the reaction solution after the termination of the synthesis reaction, and the mixture was centrifuged at 4°C and 15, 000 rpm for 5 minutes, to separate the supernatant (soluble fraction) from the precipitate (insoluble fraction). 120 µl of acetone was added to the soluble fraction, for agitation with a vortex mixer and subsequent ice cooling for 10 minutes. Subsequently, the resulting mixture was centrifuged at 4°C and 15,000 rpm for 5 minutes. After the supernatant was discarded, the precipitate was dried with a centrifuge evaporator, to which 30µl of an SDS - PAGE sample buffer was added for boiling, to prepare a sample for electrophoresis. 10µl of each of the samples for electrophoresis was subjected to Tricine-SDS-PAGE on a 20 to 15% gradient gel, which was then stained with Coomassie Brilliant Blue (CBB) to evaluate the synthetic rate of the synthesized protein domain and the solubility thereof.

The results are shown in Fig.1. In Fig.1, lane 1 shows molecular weight marker; lanes 2 to 9, samples after synthesis reaction using expression vector P011126-01 or P011126-05 in the presence or absence of zinc chloride; and lanes 10 and 11, samples after synthesis reaction with no addition of template DNA. Herein, the soluble fraction is shown on even lanes in lanes 2 through 11, while the insoluble fraction is shown in odd lanes therein. In case of protein in no relation with zinc, generally, the amount of the synthesized protein is reduced when zinc is added. In the SPL9-DBD domain in the sample after synthesis reaction using the expression vector P011126-01, however, the amount of the soluble protein was remarkably improved by addition of zinc. The SPL12-DBD domain in the sample after synthesis reaction using the expression vector P011126-05 was synthesized at an insoluble state with no zinc addition. With zinc addition, however, the solubility thereof was distinctly improved, together with the increase of the amount of the synthesized protein.

Fig.2 shows the results of the protein synthesis in the presence of various metal compounds, using the expression vector P011126-05. Herein, lane 1 shows molecular weight marker; lanes 2 to 17, samples after synthesis reaction with addition of various metal compounds; lanes 18 and 19, samples after synthesis reaction with no addition of such metal compounds, as controls; and lanes 20 and 21, samples after synthesis reaction with no addition of template DNA. The soluble fraction is shown on even lanes in the lanes 2 through 21, while the insoluble fraction is shown in odd lanes therein. The expression of the SPL12-DBD was observed mostly in the insoluble fraction in case of no addition of metal ions, while the expression thereof was observed more or less in the soluble fraction in case of addition of copper ion and iron ion. With zinc addition, the amount of the protein in the soluble fraction was distinctly increased.

### Industrial Applicability

By the method of the present invention, a ligand-binding protein can be synthesized in a cell-free protein synthesis system highly efficiently in a very simple manner. The synthesized protein highly possibly forms the accurate three dimensional structure. Thus, the protein can be used for the analysis of the structure and function thereof. By screening a novel ligand or ligand-binding protein using the method of the invention, interactions of biological molecules mainly including protein can be elucidated collectively and systematically at a high through-put. Thus, the method is very useful for the understanding of various biological phenomena at cellular level and for the development of a drug for activating or inhibiting such protein interactions.

## Claims

1. A method for producing a ligand-binding protein or a ligand-binding domain thereof, comprising a step of synthesizing said protein or domain in a cell-free protein synthesis system, wherein said cell-free protein synthesis system comprises a ligand that binds to said protein or domain.

2. The method of claim 1, wherein said ligand-binding protein or said ligand-binding domain is synthesized as a soluble protein.

3. The method of claim 1, wherein said ligand is a substance selected from the group consisting of DNAs, RNAs, nucleosides, nucleotides, proteins, peptides, amino acids, lipids, carbohydrates and derivatives thereof, alkaloids, terpenes, low molecular weight compounds, coenzymes, lowmolecular weight metal complexes, and metal ions.

4. The method of claim 1, wherein said protein is a protein or polypeptide domain of unknown function.

5. The method of claim 1, wherein said protein is a receptor, a transporter, a DNA binding protein, a protein interactive with another protein, a cytoskeletal protein, an oxidoreductase, a chromoprotein or a ligand-binding domain thereof.

6. A method for screening a ligand thatbinds to a targetprotein, comprising the steps of:
preparing a candidate ligand,
synthesizing said target protein in a cell-free protein synthesis system in the presence and absence of said candidate ligand, and
determining the synthetic rates and/or solubilities of said target protein under said respective conditions,
wherein an increase of the synthetic rate and/or solubility of said target protein in the presence of said candidate ligand indicates that said candidate ligand binds to said target protein.

7. A method for screening a ligand that binds to a target protein, comprising the steps of:
preparing a candidate ligand,
synthesizing said target protein in a cell-free protein system in the presence and absence of said candidate ligand, and
evaluating the specific activities and/or formations of higher structure of said target protein under said respective conditions,
wherein an increase or decrease of the specific activity and/or formation of the desired higher structure of said target protein in the presence of said candidate ligand indicates that said candidate ligand binds to said target protein.

8. The method of claim 6 or 7, wherein said target protein is a protein or polypeptide domain of unknown function.

9. The method of claim 6 or 7 , wherein said protein is a receptor, a transporter, a DNA binding protein, a protein interactive with another protein, a cytoskeletal protein, an oxidoreductase, a chromoprotein or a ligand-binding domain thereof.

10. The method of claim 6 or 7, wherein said ligand which binds to the target protein is an activator of said target protein.

11. The method of claim 6 or 7, wherein said ligand which binds to the target protein is an inhibitor of said target protein.

12. A method for screening a protein that binds to a target ligand, comprising the steps of:
preparing a polynucleotide coding for a candidate protein,
expressing said polynucleotide in a cell-free system in the presence and absence of said target ligand, and
measuring the expression rates of the candidate protein and/or the solubilities of the expressed candidate protein under said respective conditions,
wherein an increase of the expression rate of the candidate protein and/or solubility of said expressed candidate protein in the presence of said target ligand indicates that said candidate protein binds to said target ligand.

13. A method for screening a protein that binds to a target ligand, comprising the steps of:
preparing a polynucleotide coding for a candidate protein,
expressing said polynucleotide in a cell-free system in the presence and absence of said target ligand, and
evaluating the specific activities and/or formations of higher structure of said expressed candidate protein under said respective conditions,
wherein an increase or decrease of the specific activity and/or formation of the desired higher structure of said candidate protein by addition of said target ligand indicates that said candidate protein binds to said target ligand.

14. The method of claim 12 or 13, wherein said target ligand is a substance selected from the group consisting of DNAs, RNAs, nucleosides, nucleotides, proteins, peptides, amino acids, lipids, carbohydrates and derivatives thereof, alkaloids, terpenes, low molecular weight compounds, coenzymes, low molecular weight metal complexes, and metal ions.

15. The method of claim 12 or 13, wherein said candidate protein is a protein or polypeptide domain of unknown function.


SEQUENCE LISTING

<110> RIKEN

<120> PROCESS FOR PRODUCING LIGAND BINDING PROTEIN WITH THE USE OF CELL-FREE
PROTEIN SYNTHESIS SYSTEM AND UTILIZATION THEREOF

<130> SJW/WA/47628

<140> EP03797560.4
<141> 2003-09-09

<150> JP 2002-271375
<151> 2002-09-18

<160> 11

<170> PatentIn version 3.1

<210> 1
<211> 258
<212> DNA
<213> Arabidopsis thaliana

<220>
<223> Inventor :Kigawa, Takanori; Matsuda, Takayoshi; Aoki, Masaaki
Inventor: Yokoyama, Shigeyuki; Shinozaki, Kazuo; Seki, Motoaki

<220>
<221> CDS
<222> (1)..(258)
<223>

<400> 1
ggt cag tcg ggt cag ata cca agg tgc caa gtg gaa ggt tgt ggg atg 48
Gly Gln Ser Gly Gln Ile Pro Arg Cys Gln Val Glu Gly Cys Gly Met
1               5                   10                  15

gat cta acc aat gca aaa ggt tat tac tcg aga cac cga gtt tgt gga 96
Asp Leu Thr Asn Ala Lys Gly Tyr Tyr Ser Arg His Arg Val Cys Gly
            20                  25                  30

gtg cac tct aaa aca cct aaa gtc act gtg gct ggt atc gaa cag agg 144
Val His Ser Lys Thr Pro Lys Val Thr Val Ala Gly Ile Glu Gln Arg
        35                  40                  45

ttt tgt caa cag tgc agc agg ttt cat cag ctt ccg gaa ttt gac cta 192
Phe Cys Gln Gln Cys Ser Arg Phe His Gln Leu Pro Glu Phe Asp Leu
    50                  55                  60

gag aaa agg agt tgc cgc agg aga ctc gct ggt cat aat gag cga cga 240
Glu Lys Arg Ser Cys Arg Arg Arg Leu Ala Gly His Asn Glu Arg Arg
65                  70                  75                  80

agg aag cca cag cct gcg 258
Arg Lys Pro Gln Pro Ala
                85

<210> 2

<211>  86
<212>  PRT
<213>  Arabidopsis thaliana

<400>  2

Gly Gln Ser Gly Gln Ile Pro Arg Cys Gln Val Glu Gly Cys Gly Met
1               5                   10                  15

Asp Leu Thr Asn Ala Lys Gly Tyr Tyr Ser Arg His Arg Val Cys Gly
            20                  25                  30

Val His Ser Lys Thr Pro Lys Val Thr Val Ala [?] Ile Glu Gln Arg
        35                  40                  45

Phe Cys Gln Gln Cys Ser Arg Phe His Gln Leu Pro Glu Phe Asp Leu
    50                  55                  --

Glu Lys Arg Ser Cys Arg Arg Arg Leu Ala [?]ly His Asn Glu Arg Arg
65                  70                  75                  80

Arg Lys Pro Gln Pro Ala
                85

<210>  3
<211>  246
<212>  DNA
<213>  Arabidopsis thaliana

<220>
<221>  CDS
<222>  (1)..(246)
<223>

<400>  3
gcg att tgt tgt cag gta gac aac tgt gga gct gat tta agc aaa gtt        48
Ala Ile Cys Cys Gln Val Asp Asn Cys Gly Ala Asp Leu Ser Lys Val
1               5                   10                  15

aag gat tat cat aga cgt cat aag gtc tgt gag att cat tct aaa gct        96
Lys Asp Tyr His Arg Arg His Lys Val Cys Glu Ile His Ser Lys Ala
            20                  25                  30

act act gca ctt gtt gga gga att atg cag cgg ttt tgt cag caa tgt       144
Thr Thr Ala Leu Val Gly Gly Ile Met Gln Arg Phe Cys Gln Gln Cys
        35                  40                  45

agt agg ttt cat gtg ctt gaa gag ttt gat gag gga aag aga agt tgc       192
Ser Arg Phe His Val Leu Glu Glu Phe Asp Glu Gly Lys Arg Ser Cys
    50                  55                  60

cgt aga cgt ttg gct ggg cat aat aag cgt aga aga aaa gca aat cct       240
Arg Arg Arg Leu Ala Gly His Asn Lys Arg Arg Arg Lys Ala Asn Pro
65                  70                  75                  80

gat act                                                          246
Asp Thr

<210>  4
<211>  82
<212>  PRT
<213>  Arabidopsis thaliana

<400>  4

Ala Ile Cys Cys Gln Val Asp Asn Cys Gly Ala Asp Leu Ser Lys Val
1               5                   10                  15

Lys Asp Tyr His Arg Arg His Lys Val Cys Glu Ile His Ser Lys Ala
            20                  25                  30

Thr Thr Ala Leu Val Gly Gly Ile Met Gln Arg Phe Cys Gln Gln Cys
        35                  40                  45

Ser Arg Phe His Val Leu Glu Glu Phe Asp Glu Gly Lys Arg Ser Cys
    50                  55                  60

Arg Arg Arg Leu Ala Gly His Asn Lys Arg Arg Arg Lys Ala Asn Pro
65                  70                  75                  80

Asp Thr

<210>  5
<211>  31
<212>  DNA
<213>  Artificial Sequence

<220>
<223>  5' primer atr001003851.66FW

<400>  5
ggtgccacgc ggatccggtc agtcgggtca g                               31

<210>  6
<211>  37
<212>  DNA
<213>  Artificial Sequence

<220>
<223>   3' primer atr001003851.151RV

<400>  6
gggcggggat caatcaatca ttacgcaggc tgtggct                         37

<210> 7
<211> 228
<212> DNA
<213> Artificial Sequence

<220>
<223> 5' primer T7P-DM2-NHis

<400> 7
gctcttgtca ttgtgcttcg catgattacg aattcagatc tcgatcccgc gaaattaata 60

cgactcacta tagggagacc acaacggttt ccctctagaa ataattttgt ttaactttaa 120

gaaggagata tacatatgaa agatcatctc atccacaatg tccacaaaga ggagcacgct 180

catgcccaca acaagagctc tggcgcctcc ctggtgccac gcggatcc 228


<210> 8
<211> 178
<212> DNA
<213> Artificial Sequence

<220>
<223> 3' primer T7T-DM5

<400> 8
aatgattgat tgatccccgc ccagctgagt tggctgctgc caccgctgag caataactag 60

cataacccct tggggcctct aaacgggtct tgaggggttt tttgctgaaa ggaggaacta 120

tatccggata acctcgagct gcaggcatgc aagcttggcg aagcacaatg acaagagc 178


<210> 9
<211> 20
<212> DNA
<213> Artificial Sequence

<220>
<223> Universal primer U2

<400> 9
gctcttgtca ttgtgcttcg 20


<210> 10
<211> 35
<212> DNA
<213> Artificial Sequence

<220>
<223> 5' primer atr001004282.124FW

<400> 10
ggtgccacgc ggatccgcga tttgttgtca ggtag 35


<210> 11
<211> 44
<212> DNA

<213>  Artificial Sequence

<220>
<223>  3' primer atr001004282.205RV

<400>  11
gggcggggat caatcaatca ttaagtatca ggatttgctt ttct                        44